(19) Europäisches Patentamt
European Patent Office
Office européen des brevets

(11) **EP 3 156 427 B1**

(12) **EUROPEAN PATENT SPECIFICATION**

(45) Date of publication and mention of the grant of the patent:
**26.02.2020 Bulletin 2020/09**

(21) Application number: **15806538.3**

(22) Date of filing: **12.06.2015**

(51) Int Cl.:
*C08F 20/10* (2006.01)     *C08J 3/075* (2006.01)
*C08J 3/24* (2006.01)     *C08F 2/10* (2006.01)
*A61L 15/60* (2006.01)

(86) International application number:
**PCT/KR2015/005957**

(87) International publication number:
**WO 2015/190879 (17.12.2015 Gazette 2015/50)**

(54) **SUPER ABSORBENT RESIN**

SUPRAABSORBIERENDES HARZ

RÉSINE SUPER-ABSORBANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.06.2014 KR 20140071547**
**10.12.2014 KR 20140177736**
**11.06.2015 KR 20150082881**

(43) Date of publication of application:
**19.04.2017 Bulletin 2017/16**

(73) Proprietor: **LG Chem, Ltd.**
**Seoul 07336 (KR)**

(72) Inventors:
• **LEE, Hyemin**
**Daejeon 305-738 (KR)**
• **PARK, Sung Hyun**
**Daejeon 305-738 (KR)**
• **LEE, Myung Han**
**Daejeon 305-738 (KR)**
• **IM, Ye Hoon**
**Daejeon 305-738 (KR)**
• **KIM, Sang Eun**
**Daejeon 305-738 (KR)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
EP-A1- 1 770 113          WO-A1-2015/053538
WO-A2-01/91815          JP-A- 2012 512 739
JP-B2- 4 436 686          KR-A- 20030 068 198
KR-A- 20040 058 305     KR-A- 20140 130 034
US-A- 5 451 613

**Description**

**[TECHNICAL FIELD]**

The present invention relates to a super absorbent polymer which exhibits totally well-balanced and excellent physical properties through the optimization of a centrifuge retention capacity, an absorbency under pressure, a liquid permeability and a gel strength, and a method of its preparation.

**[BACKGROUND]**

Super absorbent polymer (SAP) is a synthetic polymer material capable of absorbing moisture from about 500 to about 1,000 times its own weight, and each manufacturer has denominated it as different names such as SAM (Super Absorbency Material), AGM (Absorbent Gel Material) or the like. Such super absorbent polymers started to be practically applied in sanitary products, and now they are widely used for preparation of various products, for example, hygiene products such as paper diapers for children, water retaining soil products for gardening, water stop materials for the civil engineering and construction, sheets for raising seedling, fresh-keeping agents for food distribution fields, materials for poultice or the like.

In most cases, these super absorbent polymers have been widely used in the field of hygienic materials such as diapers or sanitary napkins. For these applications, the super absorbent polymer should exhibit a high absorbency, it should not release the absorbed water even in the external pressure, and additionally it should well retain the shape even in a state where the volume is expanded (swelled) by absorbing water.

In particular, in order for the super absorbent polymer to exhibit more excellent physical properties and performances when used in hygienic materials such as diapers, it is necessary to excellently exhibit all of an absorption capacity, an absorption and retention capacity under pressure, a shape retaining property (liquid permeability) or the like in a good balance. Hitherto, various studies have been performed for improving the overall physical properties in a good balance.

In particular, in recent years, it is required to more greatly improve the shape retaining property (liquid permeability) of the super absorbent polymer. For the improvement of such liquid permeability, it is required to more highly exhibit the gel strength in an appropriate direction. This is because the polymer having high gel strength can well-maintain its shape even though the volume increases by absorbing water. Previously, however, a method of reliably measuring the gel strength in an appropriate direction that is directly associated with the liquid permeability has not properly been suggested, and also the super absorbent polymer which maintains an absorption capacity and an absorption and retention capacity under pressure in an appropriate level, together with excellent strength corresponding to excellent liquid permeability has not been successfully developed.

For these reasons, the super absorbent polymer that excellently exhibits all of the physical properties of the three aspects has not still been adequately developed, whereby continued studies on the improvement of physical properties of the super absorbent polymer has been desired.

KR 10-2004-0058305 A discloses an absorbent product having a gel-strength which is higher than 18 kPa.

KR 2003-0068198 A discloses water-insoluble water-swellable hydrogels coated with steric or electrostatic spacers, having an absorbency under load (AUL) (0.7 psi) of at least 20 g/g, gel strength of at least 1600 Pa, wherein the steric of cationic spacers are applied to the surface of the hydrogel in amount of from 0.05 to 5% by weight, based on the total weight of the coated hydrogels.

US 6951911 A discloses a crossed-linked polymer (A), which comprises (a) vinyl monomers selected from the group consisting of a water-soluble vinyl monomer and/or a monomer which becomes water-soluble by hydolysis and (b) a cross-linking agent as an essential component.

WO 2005/097313 A1 discloses an aqueous-liquid-absorbing agent comprising water-absorbent resin particles, wherein the water-absorbent resin particles are obtained by polymerizing a water-soluble ethylenically unsaturated monomer and having a cross-linked structure in their inside, wherein the aqueous-liquid-absorbing agent has an absorption rate (FSR) of not less than 0.2 g/g/s, a water-absorption capacity (CRC) of not less than $400 \times 10^{-7}$ cm$^3$-s/g, and a wet porosity of not less than 20%.

WO 01/91815 A2 discloses super absorbent polymer particles comprising 100 parts by weight of a water-absorbing resin surface cross-linked with 0.001 to 10 parts by weight of an oxazolinium ion, wherein the oxazolinium ion is present at surfaces of the water-absorbing resin to cross-link polymer chains at the surfaces of the water-absorbing resin.

EP 1 770 113 A1 discloses a method for manufacturing a particulate water-absorbing agent, comprising the step of surface-crosslinking, in the presence of an organic crosslinking agent which is able to react with a carboxyl group, water-absorbing resin particles obtained through a step of polymerizing an aqueous solution of unsaturated monomers.

KR 10-2014-0130034 A discloses a super absorbent polymer having a centrifugal retention capacity of 28 g/g or more, absorbency under pressure at 0.7 psi of 22 g/g or more, liquid permeability of 20 x $10^{-7}$ cm$^3$·s/g or more, and a gel strength of 7,000 to 11,000 Pa.

EP 3 020 737 A1 discloses a super absorbent polymer having a degree of decrease in absorption capacity of 0 to 1 (g/g).

**[SUMMARY OF THE INVENTION]**

The present invention provides a super absorbent polymer which exhibits totally well-balanced and excellent physical properties through the optimization of a centrifuge retention capacity, an absorbency under pressure, a liquid permeability and a gel strength, and a method of its preparation.

The present invention provides a super absorbent polymer comprising a cross-linked polymer in which a base polymer in the form of a powder obtained by polymerizing water-soluble ethylene-based unsaturated monomers having an acidic group in which at least a part thereof is neutralized is subjected to a surface crosslinking, wherein the super absorbent polymer satisfies the relationship represented by the following formula 1:

wherein the super absorbent polymer has CRC of 26 to 32 g/g, AUP of 22 to 26 g/g, a horizontal gel strength G' of 7,000 to 12,000 Pa, and SFC of 50 to 75 (•$10^{-7}$cm$^3$•s/g),

wherein the crosslinked polymer is obtained by cross-linking the surface of the base polymer in the presence of a surface crosslinking agent containing diol or polyol having 2 to 8 carbon atoms,

wherein the base polymer has a gel strength before surface crosslinking (G'; Pa) of 4500Pa or more, and

wherein the surface crosslinking reaction is performed by heating the surface crosslinking agent-added base polymer particles at 180 to 200°C for at least 20 minutes, the maximum reaction temperature of the surface crosslinking reaction is 180 to 200 °C, and the holding time at the maximum reaction temperature is 20 minutes to 50 minutes:

[Formula 1]

$$\left( \frac{AUP}{CRC} \times \frac{G'}{1000} \right)^2 > 15\ Pa^2$$

in the formula 1,

CRC represents a centrifuge retention capacity for a physiological saline solution (0.9 wt% aqueous sodium chloride solution) of the super absorbent polymer for 30 minutes, and is measured in accordance with EDANA (European Disposables and Nonwovens Association) recommended test method No. WSP 241.3,

AUP represents an absorbency under pressure for a physiological saline solution (0.9 wt% aqueous sodium chloride solution) of the super absorbent polymer under 0.7 psi for 1 hour, and is measured in accordance with EDANA recommended test method No. WSP 242.3, and

G' represents a horizontal gel strength of the super absorbent resin measured using a rheometer, after absorbing and swelling a physiological saline solution (0.9 wt% aqueous sodium chloride solution) to the super absorbent polymer for 1 hour,

wherein the horizontal gel strength G' of the super absorbent resin is measured by a method comprising the steps of:

absorbing a physiological saline solution to the super absorbent polymer to swell the super absorbent polymer; positioning the swelled super absorbent polymer between plates of a rheometer having an interval to pressurize the two plate surfaces; confirming a shear strain in the linear viscoelastic regime section where storage modulus and loss modulus are steady, while increasing the shear strain using the rheometer under vibration; and measuring the storage modulus and the loss modulus of the swelled super absorbent polymer under the confirmed shear strain, respectively, and measuring the average value of the storage modulus as a gel strength, and wherein the SFC represents a physiological saline solution (0.685 wt% aqueous sodium chloride solution) flow conductivity (•$10^{-7}$cm$^3$•s/g) for the super absorbent polymer, and is measured in accordance with the method disclosed in the specification.

The physical properties of the above super absorbent polymer can satisfy the relationship represented by the following formula 2.

[Formula 2]

$$\sqrt{\frac{(CRC+AUP)}{2} * \left(\frac{G'}{1000}\right) * SFC} > 100.$$

in the formula 2, AUP, CRC and G' are as defined in the formula 1 and SFC represents a physiological saline solution (0.685 wt% aqueous sodium chloride solution) flow conductivity ($\cdot 10^{-7} cm^3 \cdot s/g$) for the super absorbent polymer.

On the other hand, in the super absorbent polymer, the water-soluble ethylene-based unsaturated monomer may include one or more selected from the group consisting of an anionic monomer such as acrylic acid, methacrylic acid, maleic anhydride, fumaric acid, crotonic acid, itaconic acid, 2-acryloyl ethane sulfonic acid, 2-methacryloyl ethane sulfonic acid, 2-(meth)acryloyl propane sulfonic acid, or 2-(meth)acrylamide-2-methylpropane sulfonic acid, and a salt thereof; a nonionic hydrophilic monomer such as (meth)acrylamide, N-substituted (meth)acrylate, 2-hydroxyethyl(meth)acrylate, 2-hydroxypropyl(meth)acrylate, methoxypolyethyleneglycol(meth)acrylate, or polyethyleneglycol(meth)acrylate; and an unsaturated monomer containing amino group such as (N,N)-dimethylaminoethyl(meth)acrylate or (N,N)-dimethylaminopropyl(meth)acrylate, and a quaternary compound thereof.

Also, the base polymer may include a polymer wherein the above monomer is cross-linked and polymerized in the presence of one or more internal crosslinking agents selected from the group consisting of bis(meth)acrylamide having 8 to 12 carbon atoms, poly(meth)acrylate of polyol having 2 to 10 carbon atoms and poly(meth)allyl ether of polyol having 2 to 10 carbon atoms.

In addition, the super absorbent polymer may have a particle size of about 150 to 850$\mu$m).

According to the invention is also a method for preparing a super absorbent polymer of the invention comprising a cross-linked polymer according to the invention, comprising the steps:

- obtaining a base polymer by polymerizing water-soluble ethylene-based unsaturated monomers having an acidic group in which at least a part thereof is neutralized,
- subjecting the base polymer in the form of a powder to a surface crosslinking, wherein the surface crosslinking reaction is performed by heating the surface crosslinking agent-added base polymer particles at 180 to 200°C for at least 20 minutes, the maximum reaction temperature of the surface crosslinking reaction is 180 to 200 °C, and the holding time at the maximum reaction temperature is 20 minutes to 50 minutes,

wherein the surface crosslinking agent contains diol or polyol having 2 to 8 carbon atoms, and the base polymer has a gel strength before surface crosslinking (G'; Pa) of 4500Pa or more.

**[DETAILED DESCRIPTION OF THE EMBODIMENTS]**

Hereinafter, the super absorbent polymer according to specific embodiments of the invention will be described in more detail. However, they are merely presented as an example of the present invention, and will be apparent to those skilled in the art that the scope of the present invention is not limited to these embodiments and various modifications are possible according to the embodiments within the scope of the present invention.

In addition, unless stated otherwise throughout this specification, the term "comprises" or "contains" means to include any constituent element (or constituent component) without particular limitation, and it cannot be interpreted as a meaning of excluding an addition of other constituent element (or constituent component).

According to one embodiment of the invention, there is provided a super absorbent polymer comprising a cross-linked polymer in which a base polymer in the form of a powder obtained by polymerizing water-soluble ethylene-based unsaturated monomers having an acidic group in which at least a part thereof is neutralized is subjected to a surface crosslinking, wherein the super absorbent polymer satisfies the relationship represented by the following formula 1:

wherein the super absorbent polymer has CRC of 26 to 32 g/g, AUP of 22 to 26 g/g, a horizontal gel strength G' of 7,000 to 12,000 Pa, and SFC of 50 to 75 ($\cdot 10^{-7} cm^3 \cdot s/g$),
wherein the crosslinked polymer is obtained by cross-linking the surface of the base polymer in the presence of a surface crosslinking agent containing diol or polyol having 2 to 8 carbon atoms,
wherein the base polymer has a gel strength before surface crosslinking (G'; Pa) of 4500Pa or more, and
wherein the surface crosslinking reaction is performed by heating the surface crosslinking agent-added base polymer particles at 180 to 200°C for at least 20 minutes, the maximum reaction temperature of the surface crosslinking reaction is 180 to 200 °C, and the holding time at the maximum reaction temperature is 20 minutes to 50 minutes:

[Formula 1]

$$\left(\frac{AUP}{CRC} \times \frac{G'}{1000}\right)^2 > 15\, Pa^2$$

in the formula 1,

CRC represents a centrifuge retention capacity for a physiological saline solution (0.9 wt% aqueous sodium chloride solution) of the super absorbent polymer for 30 minutes, and is measured in accordance with EDANA (European Disposables and Nonwovens Association) recommended test method No. WSP 241.3,

AUP represents an absorbency under pressure for a physiological saline solution (0.9 wt% aqueous sodium chloride solution) of the super absorbent polymer under 0.7 psi for 1 hour, and is measured in accordance with EDANA recommended test method No. WSP 242.3, and

G' represents a horizontal gel strength of the super absorbent resin measured using a rheometer, after absorbing and swelling a physiological saline solution (0.9 wt% aqueous sodium chloride solution) to the super absorbent polymer for 1 hour,

wherein the horizontal gel strength G' of the super absorbent resin is measured by a method comprising the steps of:

absorbing a physiological saline solution to the super absorbent polymer to swell the super absorbent polymer; positioning the swelled super absorbent polymer between plates of a rheometer having an interval to pressurize the two plate surfaces; confirming a shear strain in the linear viscoelastic regime section where storage modulus and loss modulus are steady, while increasing the shear strain using the rheometer under vibration; and measuring the storage modulus and the loss modulus of the swelled super absorbent polymer under the confirmed shear strain, respectively, and measuring the average value of the storage modulus as a gel strength, and wherein the SFC represents a physiological saline solution (0.685 wt% aqueous sodium chloride solution) flow conductivity ($\cdot 10^{-7} cm^3 \cdot s/g$) for the super absorbent polymer, and is measured in accordance with the method disclosed in the specification.

Also, the physical properties of the super absorbent polymer according to one embodiment can satisfy the relationship represented by the following formula 2.

[Formula 2]

$$\sqrt{\frac{(CRC+AUP)}{2} * \left(\frac{G'}{1000}\right) * SFC} > 100$$

in the formula 2, AUP, CRC and G' are as defined in the formula 1 and SFC represents a physiological saline solution (0.685 wt% aqueous sodium chloride solution) flow conductivity ($\cdot 10^{-7} cm^3 \cdot s/g$) for the super absorbent polymer.

The present inventors have conducted researches to further improve physical properties of the super absorbent polymer in a good balance. As a result, the inventors have found that the super absorbent polymer having optimized internal and surface crosslinking structures can be obtained by optimizing the conditions of the production process of the super absorbent polymer to be described later, for example, type and content of an internal crosslinking agent to be described later, surface crosslinking conditions, and the like.

It has been found that the super absorbent polymer having a crosslinking structure thus optimized can excellently express and maintain a centrifuge retention capacity (CRC) reflecting its basic water absorption capacity, together with an absorbency under pressure (AUP) reflecting an absorption and retention capacity under pressure.

In addition, it has been found that, as the super absorbent polymer excellently exhibits the horizontal gel strength (G'), it is excellent in the characteristics of retaining its shape even after water is absorbed and swelled in sanitary materials, and as a result, liquids can well flow through such super absorbent polymer, thus exhibiting excellent liquid permeability.

As the super absorbent polymer exhibits totally well-balanced and excellent physical properties such as a centrifuge retention capacity (CRC), an absorbency under pressure (AUP), a liquid permeability (physiological saline solution flow conductivity; SFC) and a horizontal gel strength (G'), it has been found that the formula 1 and the relationship derived therefrom satisfy the values of about 15 Pa$^2$ or more, or about 15 to 100 Pa$^2$, or about 25 to 95 Pa$^2$. In addition, it has been found that the formula 2 and the relationship derived therefrom satisfy the values of 100 or more, or about 100 to about 150, preferably about 115 to about 145.

Thus, as the super absorbent polymer of one embodiment excellently exhibits all of the overall physical properties such as an absorption capacity, an absorption and retention capacity under pressure and a shape retaining property (liquid permeability) in a good balance, it allows the expression of very excellent performances when applied to various sanitary materials such as diapers and sanitary napkins, and furthermore it provides a next generation diaper to which the ultra-thin technique is applied.

The centrifuge retention capacity (CRC) for the physiological saline solution can be calculated by the following calculation equation 1 after absorbing the super absorbent polymer in a physiological saline solution over a period of 30 minutes.

[Calculation Equation 1]

$$CRC(g/g) = \{[W_2(g) - W_1(g) - W_0(g)]/W_0(g)\}$$

in the calculation equation 1,

$W_0(g)$ is an initial weight(g) of the super absorbent polymer, $W_1(g)$ is a weight of the device not including the super absorbent polymer, measured after soaking the same in a physiological saline solution for 30 minutes and dehydrating the same by using a centrifuge at 250 G for 3 minutes, and $W_2(g)$ is the weight of the device including the super absorbent polymer, measured after soaking the same in a physiological saline solution at room temperature for 30 minutes, and then dehydrating the same by using a centrifuge at 250 G for 3 minutes.

The absorbency under pressure (AUP) can be calculated by the following calculation equation 2 after absorbing the super absorbent polymer in a physiological saline solution under pressure of about 0.7 psi over a period of 1 hour.

[Calculation Equation 2]

$$AUP(g/g) = [W_4(g) - W_3(g)]/ W_0(g)$$

in the calculation equation 2,

$W_0(g)$ is an initial weight(g) of the super absorbent polymer, $W_3(g)$ is the total sum of a weight of the super absorbent polymer and a weight of the device capable of providing a load to the super absorbent polymer, and $W_4(g)$ is the total sum of a weight of the super absorbent polymer and a weight of the device capable of providing a load to the super absorbent polymer, after absorbing a physiological saline solution to the super absorbent polymer under a load (0.7 psi) for 1 hour.

As the super absorbent polymer of one embodiment exhibits the centrifuge retention capacity (CRC) and the absorbency under pressure (AUP) within the above-described range, the basic absorption capacity and the absorption and retention capacity under pressure of the super absorbent polymer are excellently expressed and thus the super absorbent polymer can be suitably used for various sanitary materials.

The horizontal gel strength G' is a physical property of the super absorbent polymer newly measured by the present inventors, and can better reflect excellent liquid permeability under the environments of actually using water absorbent polymer. That is, conventionally the vertical gel strength of the super absorbent polymer has been measured while providing a force to the super absorbent polymer in the vertical direction. However, the liquid permeability of the super absorbent polymer has been found to be highly relevant depending on whether to exhibit excellent shape retaining property and high gel strength, irrespective of the force provided in the horizontal direction when the super absorbent resin was contained in the sanitary materials such as diapers.

Accordingly, the present inventors has designed a method of measuring a new parameter that can more effectively reflect and predict an excellent liquid permeability under the environment of actually using such super absorbent polymer. Herein, the parameter is just the horizontal gel strength G'.

The super absorbent polymer of one embodiment that the horizontal gel strength G' satisfies the range described above exhibits excellent liquid permeability together with excellent CRC and AUP as already described above. Therefore, it has been confirmed that this super absorbent polymer can be preferably used for the hygiene materials such as diapers.

This horizontal gel strength G' can be measured by a method comprising the following respective steps by using a commercialized rheometer, after a physiological saline has been absorbed to the super absorbent polymer for 1 hour.

> 1) a step of absorbing a physiological saline solution to the super absorbent polymer to swell the super absorbent polymer;
> 2) a step of positioning the swelled super absorbent polymer between plates of a rheometer having an interval to pressurize the two plate surfaces;
> 3) a step of confirming a shear strain in the linear viscoelastic regime section where storage modulus and loss modulus are steady, while increasing the shear strain using the rheometer under vibration; and
> 4) a step of measuring the storage modulus and the loss modulus of the swelled super absorbent polymer under the confirmed shear strain, respectively, and measuring the average value of the storage modulus as a gel strength.

The super absorbent polymer that the horizontal gel strength G' measured by the above method meets the range described above can satisfy the relationship of the formula 1 already described for the relationship between the CRC and AUP, and also it can satisfy the relationship of the formula 2 in the relationship of CRC, AUP and SFC. Accordingly, since the overall physical properties required for the super absorbent polymer are excellently exhibited in a good balance, the super absorbent polymer can be very suitably used for sanitary materials such as diapers.

The saline flow conductivity (SFC) can be measured and calculated according to the method well-known to those skilled in the art, for example, the method disclosed in column 6 [0184] to [0189] of US Patent Application Publication No. 2009-0131255. Further, when calculating the value of the left side of the formula 2 described above on the basis of the measured values of the SFC, the value can be calculated by substituting for the left side of the formula 2 except for the scale and unit section of "$\cdot 10^{-7} cm^3 \cdot s/g$".

On the other hand, the super absorbent polymer of one embodiment can be typically obtained by polymerizing a water-soluble ethylene-based unsaturated monomers having an acidic group in which at least a part thereof is neutralized, such as an acrylic acid in which at least a part of carboxylic acid is neutralized with sodium salt or the like. More specifically, the super absorbent polymer can be obtained by crosslinking and polymerizing the above monomers in the presence of an internal crosslinking agent to obtain a base polymer in a powder form, and then surface-crosslinking the base polymer in the presence of a predetermined surface crosslinking agent to produce a cross-linked polymer.

Such super absorbent polymer can be in the form of a cross-linked polymer from a water-soluble ethylene-based unsaturated monomers such as an acrylic acid having an internal crosslinking structure. The surface of these cross-linked polymer particles is further cross-linked and thus the degree of crosslinking can be more improved. Although described in more detail below, the super absorbent polymer of one embodiment has more suitable internal and surface crosslinking structures through the optimization of the internal crosslinking process or the surface crosslinking process, and thus it can exhibit the overall physical properties of one embodiment described above.

In the super absorbent polymer, the above water-soluble ethylene-based unsaturated monomers may include one or more selected from the group consisting of an anionic monomer such as acrylic acid, methacrylic acid, maleic anhydride, fumaric acid, crotonic acid, itaconic acid, 2-acryloyl ethane sulfonic acid, 2-methacryloyl ethane sulfonic acid, 2-(meth)acryloyl propane sulfonic acid, or 2-(meth)acrylamide-2-methylpropane sulfonic acid, and a salt thereof; a nonionic hydrophilic monomer such as (meth)acrylamide, N-substituted (meth)acrylate, 2-hydroxyethyl(meth)acrylate, 2-hydroxypropyl(meth)acrylate, methoxypolyethyleneglycol(meth)acrylate, or polyethyleneglycol(meth)acrylate; and an unsaturated monomer containing amino group such as (N,N)-dimethylaminoethyl(meth)acrylate or (N,N)-dimethylaminopropyl(meth)acrylate, and a quaternary compound thereof. Among them, acrylic acid or its salts, for example, the alkali metal salts such as an acrylic acid in which at least a part of acrylic acid is neutralized and/or a sodium salt thereof. By using these monomers, the preparation of the super absorbent polymer having excellent physical properties become possible. In the case of using the alkali metal salt of acrylic acid as a monomer, it is possible to use acrylic acid after neutralizing the same with a basic compound such as sodium hydroxide (NaOH).

Further, the internal crosslinking agent for crosslinking and polymerizing these monomers used herein may include one or more selected from the group consisting of bis(meth)acrylamide having 8 to 12 carbon atoms, poly(meth)acrylate of polyol having 2 to 10 carbon atoms, and poly(meth)allyether of polyol having 2 to 10 carbon atoms. More specifically, as the internal crosslinking agent, one or more poly(meth)acrylates of polyols selected from the group consisting of polyethylene glycol di(meth)acrylate, polypropyleneoxy di(meth)acrylate, glycerol diacrylate, glycerol triacrylate and trimethylol triacrylate can be suitably used. Among them, it has been found that by using an internal crosslinking agent such as the polyethylene glycol di(meth)acrylate, the base polymer and the super absorbent polymer wherein the internal cross-linked structure is more optimized can be obtained, whereby the super absorbent polymer satisfying the physical properties according to one embodiment can be more suitably obtained.

More specifically, by using a particular internal crosslinking agent in a certain amount, the base polymer having a gel strength before surface crosslinking (G'; Pa) of about 4500Pa or more, or about 4600Pa or more, or about 4600 to 7000Pa can be obtained. As the surface cross-linking step proceeds under certain conditions which will be described later, it is possible to obtain the super absorbent polymer of one embodiment satisfying the relationship of the formulae 1 and/or 2.

For example, the certain internal crosslinking agent can be used in a ratio of about 0.005 mol or more, or about 0.005 to 0.1 mol, or about 0.005 to 0.05 mol (or about 0.3 or more parts by weight, or 0.3 to 0.6 parts by weight relative to 100 parts by weight of acrylic acid), based on 1 mol of non-neutralized acrylic acid contained in the monomer. The base polymer having a gel strength (G'; Pa) before surface crosslinking of about 4500Pa or more can be suitably obtained according to the range of the content of the internal crosslinking agent. By using the internal crosslinking agent, the super absorbent polymer satisfying the relationship of the formulae 1 and/or 2 can be obtained.

After polymerizing the monomers using the internal crosslinking agent, processes such as drying, pulverizing and classifying are performed to obtain a base polymer in the form of a powder. Through the processes such as the pulverizing and classifying, the base polymer and the super absorbent polymer obtained therefrom are suitably prepared and provided so as to have a particle size of about 150 to 850 $\mu$m. More specifically, at least about 95% by weight of the base polymer and the super absorbent polymer obtained therefrom have a particle size of about 150 to 850$\mu$m, and fine particles having a particle size of less than about 150 $\mu$m can be less than about 3% by weight, or less than about 1% by weight.

By adjusting the particle size distribution of the base polymer and the super absorbent polymer within the preferred range, the super absorbent polymer of one embodiment can exhibit the physical properties already mentioned above and more excellent liquid permeability.

In addition, the super absorbent polymer of one embodiment includes a cross-linked polymer obtained by subjecting the base polymer to a surface crosslinking. This surface crosslinking is performed in the presence of a surface crosslinking agent including diol or polyol having 2 to 8 carbon atoms. As the surface crosslinking is performed in the presence of the surface crosslinking agent, the super absorbent polymer wherein the surface crosslinking structure is optimized can be suitably obtained. This super absorbent polymer can more suitably satisfy the overall physical properties already mentioned above, such as the relationship of the formulae 1 and 2.

More suitable examples of the diol or polyol having 2 to 8 carbon atoms that can be used as this surface crosslinking agent can include 1,3-propanediol, 1,6-hexanediol, ethylene glycol, propylene glycol, 1,2-hexanediol, 1,3-butanediol, 2-methyl-1,3-propanediol, 2,5-hexanediol, 2-methyl-1,3-pentanediol, 2-methyl-2,4-pentanediol or the like, and it can be used in combination of two or more selected among them.

In addition, as the surface crosslinking proceeds by addition of polyvalent metal cations together with the above surface crosslinking agent, the surface crosslinking structure of the super absorbent polymer can be further optimized. It is expected that this is because the metal cation forms a carboxyl group (COOH) and a chelate of the super absorbent polymer to thereby further reduce the crosslinking distance.

On the other hand, hereinafter, the method for preparing the super absorbent polymer of one embodiment described above, more specifically, the super absorbent polymer satisfying the above-described various physical properties, such as the relationship of the formulae 1 and 2, will be described in more detail according to respective steps. However, with regard to the monomers, internal crosslinking agent, surface crosslinking agent and particle size distribution already described above, duplicating explanation thereon will be omitted, and the remaining configuration and condition will be described in detail according each step of the process.

The method for preparing the super absorbent polymer may comprise the steps of: forming a hydrogel polymer by carrying out a thermal polymerization or photo polymerization of a monomer composition including a water-soluble ethylene-based unsaturated monomer, an internal crosslinking agent and a polymerization initiator; drying the hydrogel polymer; pulverizing and classifying the dried polymer to form a base polymer; and subjecting the base polymer to a surface crosslinking. In particular, in the above preparation method, as the internal crosslinking agent, the surface crosslinking agent and the particle size distribution as already described above are applied and also the contents of the internal and/or surface crosslinking agents to be described later, or process conditions such as crosslinking/polymerizing process and surface crosslinking process using these contents are optimized, the super absorbent polymer satisfying the overall physical properties such as the relationship of the formulae 1 and 2 can be obtained.

In the above preparation method, the monomer composition includes a water-soluble ethylene-based unsaturated monomer, an internal crosslinking agent and a polymerization initiator, and the types of the monomers are the same as those already described above.

Further, in the above composition, the concentration of the water-soluble ethylene-based unsaturated monomer may be about 20 to about 60% by weight, or about 40 to about 50% by weight based on the entire monomer composition including the respective raw materials and solvents described above, and it may be controlled to be an adequate concentration in consideration of the polymerization time, the reaction conditions or the like. However, when the concentration of the monomer is too low, the yield of the super absorbent polymer is low and there may be a problem with economics. By contrast, when the concentration is too high, there may be problems on the process that some of the monomers may be deposited or the pulverizing efficiency of the prepared hydrogel polymer appears to be low in the pulverizing process, and thus the properties of the super absorbent polymer may decrease.

Further, the polymerization initiator is not particularly limited as long as it is what is generally used in the preparation of the super absorbent polymer.

Specifically, the polymerization initiator may include a thermal polymerization initiator or a photo polymerization initiator by UV irradiation, according to the polymerization method. However, even in the case of photo polymerization method, a thermal polymerization initiator may be additionally included because a certain amount of heat is generated by the irradiation of UV ray and the like and a certain amount of heat is generated according to the progress of the exothermic polymerization reaction.

The photo polymerization initiator can be used without limitation in the constitution as long as it is a compound which can form a radical by a light such as an UV ray.

The photo polymerization initiator used herein may include, for example, one or more initiators selected from the group consisting of a benzoin ether, a dialkyl acetophenone, a hydroxyl alkylketone, a phenyl glyoxylate, a benzyl dimethyl ketal, an acyl phosphine, and an $\alpha$-aminoketone. Meanwhile, as the specific example of the acyl phosphine, commercialized lucirin TPO, namely, 2,4,6-trimethyl-benzoyl-trimethyl phosphine oxide may be used. More various photo polymerization initiators are well disclosed in "UV Coatings: Basics, Recent Developments and New Application" written by Reinhold Schwalm, (Elsevier, 2007), p 115, however the example of the photo polymerization initiator is not limited thereto.

The photo polymerization initiator may be included in the concentration of about 0.01% to about 1.0% by weight based on the monomer composition. When the concentration of the photo polymerization initiator is too low, the polymerization rate may become slow, and when the concentration of the photo polymerization initiator is too high, the molecular weight of the super absorbent polymer becomes small and the physical properties may become uneven.

And, as the thermal polymerization initiator, one or more initiators selected from the group consisting of a persulfate-based initiator, an azo-based initiator, hydrogen peroxide, and ascorbic acid may be used. Specific examples of the persulfate-based initiator may include sodium persulfate ($Na_2S_2O_8$), potassium persulfate ($K_2S_2O_8$), ammonium persulfate (($NH_4)_2S_2O_8$), and the like; and examples of the azo-based initiator include 2,2-azobis(2-amidinopropane)dihydrochloride, 2,2-azobis-(N, N-dimethylene)isobutyramidine dihydrochloride, 2-(carbamoylazo)isobutylonitril, 2,2-azo-bis[2-(2-imidazolin-2-yl)propane]dihydrochloride, 4,4-azobis-(4-cyanovaleric acid) or the like. More various thermal polymerization initiators are well disclosed in "Principle of Polymerization" written by Odian, (Wiley, 1981), p 203, however the example of the thermal polymerization initiator is not limited thereto.

The thermal polymerization initiator may be included in the concentration of about 0.001% to about 0.5% by weight based on the monomer composition. When the concentration of the thermal polymerization initiator is too low, the additional thermal polymerization hardly occurs and the effect resulting from the addition of the thermal polymerization initiator may be poor, and when the concentration of the thermal polymerization initiator is too high, the molecular weight of the super absorbent polymer becomes small and the physical properties may become uneven.

In addition, the types of the internal crosslinking agent contained together with the monomer composition are the same as those already described above. Such internal crosslinking agent may be included in a concentration of about 0.01% to about 0.5% by weight based on the monomer composition and thus can cross-link the prepared polymer. Further, as already described above, the internal crosslinking agent can be used in a ratio of about 0.005 mol or more, or about 0.005 to 0.1 mol, or about 0.005 to 0.05 mol (or about 0.3 or more parts by weight, or 0.3 to 0.6 parts by weight relative to 100 parts by weight of acrylic acid), based on 1 mol of non-neutralized acrylic acid contained in the certain internal crosslinking agent. As the internal crosslinking agent is used within such content range, it can suitably satisfy the range of the gel strength before surface crosslinking described above. By using the above, the super absorbent polymer more suitably satisfying the physical properties according to one embodiment can be obtained.

In addition, the monomer composition may further include additives such as a thickener, a plasticizer, a preservation stabilizer, an antioxidant, and so on, as needed.

The monomer composition may be prepared in the form of solution wherein the raw materials such as the water-soluble ethylene-based unsaturated monomer, the photo polymerization initiator, the thermal polymerization initiator, the internal crosslinking agent, and the additives are dissolved in a solvent.

At this time, the above-described solvents can be used without limitation in the constitution as long as they are those which can dissolve said components. For example, one or more solvents selected from the group consisting of water, ethanol, ethyleneglycol, diethyleneglycol, triethyleneglycol, 1,4-butanediol, propyleneglycol, ethyleneglycol monobutylether, propyleneglycol monomethylether, propyleneglycol monomethylether acetate, methylethylketone, acetone, methylamylketone, cyclohexanone, cyclopentanone, diethyleneglycol monomethylether, diethyleneglycol ethylether, toluene, xylene, butylolactone, carbitol, methylcellosolve acetate, and N,N-dimethyl acetamide, and so on may be used alone or in combination.

The solvent may be included in the residual quantity excluding the components disclosed above based on the total content of the monomer composition.

Meanwhile, the method of preparing a hydrogel polymer by subjecting such monomer composition to the thermal polymerization or photo polymerization can be used without limitation in the constitution as long as it is a method generally used in the polymerization.

Specifically, the polymerization method is largely classified into the thermal polymerization and the photo polymerization according to the polymerization energy source. Usually, the thermal polymerization may be carried out in the reactor like kneader equipped with agitating spindles, and the photo polymerization may be carried out in the reactor equipped with movable conveyor belt, however the polymerization methods disclosed above are just the examples, and the present invention is not limited to the polymerization methods disclosed above.

As an example, the hydrogel polymer obtained by subjecting to the thermal polymerization in the reactor like kneader equipped with the agitating spindles disclosed above by providing hot air thereto or heating the reactor may have the size of centimeters or millimeters when it is discharged from the outlet of the reactor, according to the types of the agitating spindles equipped in the reactor. Specifically, the size of the obtained hydrogel polymer can be variously shown according to the concentration of the monomer composition fed thereto, the feeding speed, and the like, and the hydrogel polymer of which the weight average particle diameter is about 2 to 50 mm can be generally obtained.

Further, as described above, when the photo polymerization is carried out in a reactor equipped with a movable conveyor belt, the hydrogel polymer typically obtained may be a hydrogel polymer in a sheet-type having a width of the belt. In this case, the thickness of the polymer sheet may vary according to the concentration of the monomer composition fed thereto and the feeding speed, and the polymer sheet is preferably controlled to have a thickness of about 0.5 to about 5 cm. If the monomer composition is fed so that the thickness of the sheet-type polymer becomes too thin, the production efficiency becomes low, which is not preferred. If the thickness of the sheet-type polymer exceeds 5 cm, the polymerization reaction may not uniformly occur throughout the thickness of the polymer due to the excessively high thickness.

In this case, the hydrogel polymer thus obtained by the method may have typically a water content of about 40 to about 80% by weight. Meanwhile, the term "water content" as used herein means a water content in the total weight of the hydrogel polymer, which is obtained by subtracting the weight of the dried polymer from the weight of the hydrogel polymer. Specifically, the water content is defined as a value calculated by measuring the weight loss according to evaporation of water in the polymer during the drying process of increasing the temperature of the polymer with infrared heating. At this time, the water content is measured under the drying conditions which are determined as follows; the temperature is increased from room temperature to about 180°C, then the temperature is maintained at 180°C, and the total drying time is set to 20 minutes, including 5 minutes for the temperature rising step.

Next, the step of drying the hydrogel polymer thus obtained is performed.

If necessary, a coarsely pulverizing step may be performed before the drying step, in order to increase the efficiency of the drying step.

In this case, a pulverizing device used herein may include, but the constitution is not limited, any one selected from the group consisting of a vertical pulverizing device, a turbo cutter, a turbo grinder, a rotary cutter mill, a cutter mill, a disc mill, a shred crusher, a crusher, a chopper, and a disc cutter, but is not limited thereto.

In this case, the coarsely pulverizing step may be performed so that the hydrogel polymer has a particle size of about 2 to about 10 mm.

To pulverize the polymer to have a particle size of less than 2 mm is technically not easy due to a high water content of the hydrogel polymer, and a phenomenon of agglomeration may occur between the pulverized particles. Meanwhile, if the polymer is pulverized to have a particle size of larger than 10 mm, the effect of increasing the efficiency in the subsequent drying step may be insignificant.

The hydrogel polymer coarsely pulverized as above or immediately after polymerization without the coarsely pulverizing step is subjected to a drying step. At this time, the drying temperature of the drying step may be about 150 to about 250°C. When the drying temperature is less than 150°C, there is a concern that the drying time becomes excessively long or the physical properties of the super absorbent polymer finally formed may be deteriorated, and when the drying temperature is higher than 250°C, only the surface of the polymer is dried, and thus there is a concern that fine powder may be generated during the subsequent pulverization process and the physical properties of the super absorbent polymer finally formed may be deteriorated. Therefore, the drying process may be preferably performed at a temperature of about 150 to about 200°C, and more preferably about 160 to about 180°C.

Meanwhile, the drying step may be carried out for about 20 to about 90 minutes, in consideration of the process efficiency, but is not limited thereto.

Furthermore, any known drying method may be selected and used in the drying step without limitation in the constitution if it can be generally used for drying the hydrogel polymer. Specifically, the drying step may be carried out by a method of supplying hot air, irradiating infrared rays, irradiating microwaves, irradiating ultraviolet rays or the like. When the drying step as above is finished, the water content of the polymer may be about 0.1 to about 10% by weight.

Next, the dried polymer obtained from the drying step is subjected to a pulverization step.

The polymer powder obtained from the pulverization step may have a particle size of about 150 to about 850 $\mu$m. Specific examples of a milling device that can be used to achieve the above particle size may include a pin mill, a hammer mill, a screw mill, a roll mill, a disc mill, a jog mill or the like, but the present invention is not limited thereto.

In order to properly control the physical properties of the super absorbent polymer powder finally manufactured after the pulverization step, a separate classifying step can be performed according to the particle sizes of the polymer powders obtained from the pulverization. Preferably, a polymer having a particle size of about 150 to about 850 $\mu$m is classified and only particle having such particle size is subjected to the surface crosslinking reaction and finally, it is commercialized. The particle size distribution of the base polymer obtained through such process has been described above, and thus more specific description thereon will be omitted.

The horizontal gel strength (G') of the above-described base polymer before surface crosslinking can satisfy the range of about 4500Pa or more, or about 4600Pa or more, or about 4600 to 7000Pa. When the horizontal gel strength of the base polymer before surface crosslinking is within the above range, the super absorbent polymer, which is prepared through surface crosslinking reaction to be described later, can satisfy the above physical properties, or the relationship of the formulae 1 and 2.

On the other hand, after obtaining the base polymer through the pulverizing and classifying processes, the super absorbent polymer of one example can be prepared through the surface crosslinking process. The types of the surface crosslinking agents usable in the surface crosslinking process have been described above, and thus more specific description thereon will be omitted.

With regard to the method of adding the surface crosslinking agent to the base polymer, there is no limitation in the constitution. For example, a method of adding and mixing the surface crosslinking agent and the base polymer in a reactor, a method of spraying the surface crosslinking agent onto the base polymer, or a method of continuously feeding the base polymer and the surface crosslinking agent to a mixer which is continuously operated, or the like, may be used.

During the addition of the surface crosslinking agent, water and methanol can be further mixed and added together. When adding water and methanol, there is an advantage that the surface crosslinking agent can be evenly dispersed in the base polymer. In this case, the content of water and methanol to be added can be applied by adjusting the addition rate with respect to 100 parts by weight of the base polymer, for the purpose of inducing the uniform dispersion of a surface crosslinking agent, preventing the phenomenon of aggregation of the base polymer powder and at the same time optimizing the penetrated depth from the surface.

The surface crosslinking reaction is allowed to occur by heating the surface crosslinking agent-added base polymer particles at about 180 to 200°C for at least 20 minutes. In particular, for the production of super absorbent polymer capable of more adequately satisfying the physical properties according to one embodiment, the conditions of the surface crosslinking process are as follows: the maximum reaction temperature is about 180 to 200 °C, and the holding time at the maximum reaction temperature is 20 minutes, to 50 minutes. In addition, the time of temperature rise during the starting of the initial reaction, particularly, the initial mixing temperature of the reaction solution containing the surface crosslinking agent with the base polymer, that is, the temperature of about 60°C or more, or about 100 to 170°C until it reach the maximum reaction temperature, can be controlled to at least about 10 minutes, or more than 10 minutes and not more than 60 minutes, or more than about 10 minutes and not more than 40 minutes. It has been found that the super absorbent polymer suitably satisfying the physical properties according to one embodiment can be prepared by satisfying the conditions of the surface crosslinking process described above.

A means for raising the temperature for surface crosslinking reaction is not particularly limited. Heating may be performed by providing a heating medium or by directly providing a heat source. In this case, the type of the heating medium applicable herein may be a hot fluid such as steam, hot air, hot oil, or the like, but the present invention is not limited thereto. The temperature of the heating medium provided may be properly controlled, considering the means of the heating medium, the heating rate, and the target temperature. Meanwhile, as the heat source provided directly, an electric heater or a gas heater may be used, but the present invention is not limited to these examples.

The super absorbent polymer prepared by the preparation method described above satisfies excellent horizontal gel strength and liquid permeability without reducing the physical properties such as a centrifuge retention capacity and an absorbency under pressure and thus can satisfy the relationship of the formulae 1 and 2, and exhibit excellent overall physical properties that can be suitably used in the sanitary materials such as diapers.


**[ADVANTAGEOUS EFFECTS]**


According to the present invention, the conditions of the production process of the super absorbent polymer, for example the internal and surface crosslinking processes are controlled, thereby excellently expressing and maintaining a centrifuge retention capacity (CRC) reflecting its basic absorption capacity, together with an absorbency under pressure (AUP) reflecting an absorption and retention capacity under pressure and also excellently exhibiting the horizontal gel strength (G'). Therefore, the super absorbent polymer having excellent characteristics of retaining its shape even after water is absorbed and swelled in sanitary materials, and also exhibiting excellent liquid permeability can be provided.

Such super absorbent polymer can exhibit totally well-balanced and excellent physical properties such as a centrifuge retention capacity (CRC), an absorbency under pressure (AUP), a liquid permeability (physiological saline solution flow conductivity; SFC) and a horizontal gel strength (G'), thereby satisfying he relationship of the formulae 1 and 2 described above.

Thus, as the super absorbent polymer according to an embodiment of the invention exhibits well-balanced and excellent overall physical properties such as an absorption capacity, an absorption and retention capacity under pressure and a shape retaining property (liquid permeability), it allows the expression of very excellent performances when applied to various sanitary materials such as diapers and sanitary napkins, and furthermore it provides a next generation diaper to which the ultra-thin technique is applied.

**[EXAMPLES]**

Hereinafter, the preferred Examples are provided for better understanding of the invention. However, these Examples are given for illustrative purposes only and not intended to limit the scope of the present invention.
In the Examples and Comparative Examples below, the physical properties of the respective super absorbent polymers were measured and evaluated by the following methods.

**(1) Evaluation of Particle Size**

The particle sizes of the base polymer and the super absorbent polymer used in Examples and Comparative Examples were measured in accordance with EDANA (European Disposables and Nonwovens Association) recommended test method No. WSP 220.3.

**(2) CRC (Centrifuge Retention Capacity)**

For the absorbent polymer prepared in Examples and Comparative Examples, the centrifuge retention capacity (CRC) by absorption capacity under a non-loading condition was measured in accordance with EDANA (European Disposables and Nonwovens Association) recommended test method No. WSP 241.3.
That is, after uniformly inserting $W_0(g)$ (about 0.2 g) of each polymer obtained in Examples and Comparative Examples in a nonwoven fabric-made bag and sealing the same, it was soaked in a physiological saline solution composed of 0.9 wt% aqueous sodium chloride solution at room temperature for 30 minutes. After dehydrating the same by using a centrifuge at 250 G for 3 minutes, the weight $W_2(g)$ of the bag was measured. Further, after carrying out the same operation without using the base polymer, the weight $W_1(g)$ of the bag was measured.
CRC (g/g) was calculated by using the obtained weight values according to the following calculation equation 1, thereby confirming the centrifuge retention capacity.

$$[\text{Calculation Equation 1}]$$

$$CRC(g/g) = \{[W_2(g) - W_1(g) - W_0(g)]/W_0(g)\}$$

in the calculation equation 1,

$W_0(g)$ is an initial weight(g) of the super absorbent polymer,
$W_1(g)$ is a weight of the device not including the super absorbent polymer, measured after soaking the same in a physiological saline solution for 30 minutes and dehydrating the same by using a centrifuge at 250 G for 3 minutes, and
$W_2(g)$ is a weight of the device including the super absorbent polymer, measured after soaking the same in a physiological saline solution at room temperature for 30 minutes, and then dehydrating the same by using a centrifuge at 250 G for 3 minutes.

**(3) Absorbency under Pressure (AUP)**

For the absorbent polymer prepared in Examples and Comparative Examples, the absorbency under pressure was measured in accordance with EDANA (European Disposables and Nonwovens Association) recommended test method No. WSP 242.3.
First, a 400 mesh stainless steel net was installed in the cylindrical bottom of a plastic having an internal diameter of 60 mm. $W_0$(g, 0.90 g) of the absorbent polymers prepared in Examples 1-6 and Comparative Examples 1-3 were uniformly scattered on the steel net under conditions of temperature of $23\pm2°C$ and relative humidity of 45%, and a piston which can provide a load of 4.83 kPa (0.7 psi) uniformly was put thereon. The external diameter of the piston was slightly smaller than 60 mm, there was no gap between the cylindrical internal wall and the piston, and the jig-jog of the cylinder was not interrupted. At this time, the weight $W_3(g)$ of the device was measured.

After putting a glass filter having a diameter of 125 mm and a thickness of 5 mm in a Petri dish having a diameter of 150 mm, a physiological saline solution composed of 0.90 wt% of sodium chloride was poured in the dish until the surface level became equal to the upper surface of the glass filter. A sheet of filter paper having a diameter of 120 mm was put thereon. The measuring device was put on the filter paper and the solution was absorbed under a load for about 1 hour. After 1 hour, the weight $W_4$(g) was measured after lifting the measuring device up.

Using the respective mass fractions thus obtained, AUP(g/g) was calculated according to the following Calculation Equation 2, thereby confirming the absorbency under pressure.

[Calculation Equation 2]

$$AUP(g/g) = [W_4(g) - W_3(g)]/ W_0(g)$$

in the calculation equation 2,

$W_0$(g) is an initial weight(g) of the super absorbent polymer,

$W_3$(g) is the total sum of a weight of the super absorbent polymer and a weight of the device capable of providing a load to the super absorbent polymer, and

$W_4$(g) is the total sum of a weight of the super absorbent polymer and a weight of the device capable of providing a load to the super absorbent polymer, after absorbing a physiological saline solution to the super absorbent polymer under a load (0.7 psi) for 1 hour,

**(4) Gel Strength (G')**

For the absorbent polymers prepared in Examples 1-6 and Comparative Examples 1-3, the horizontal gel strength was measured.

First, the absorbent polymer samples (30-50 mesh) prepared in Examples 1-6 and Comparative Examples 1-3 were sieved off and 0.5 g of the samples were weighed. The weighed samples were sufficiently swelled in 50 g of a physiological saline solution for 1 hour. After that, the solvent not absorbed therein was removed by using an aspirator for 4 minutes, and the solvent left on the surface of the same was evenly distributed and wiped once with a filter paper.

2.5 g of the swelled super absorbent polymer was loaded between two plates (parallel plates with a 25 mm diameter, a lower plate thereof having a wall with a 2 mm height for preventing the sample from leaking) of the rheometer, and the gap (1 mm) between the plates was adjusted. At this time, the gap between the parallel plates was properly adjusted by pressing the plates with a force of 3 N so that the swelled sample contacted evenly at the face of the plates.

A linear viscoelastic regime section of strain where the storage modulus (G') and the loss modulus (G") were steady was found by using the rheometer while increasing the shear strain at a 10 rad/s oscilation frequency. Generally, in the case of a swelled super absorbent polymer, a strain of 0.1% is imparted in the liner viscoelastic regime section.

The storage modulus and the loss modulus of the swelled super absorbent polymer was measured by using the strain value of the linear viscoelastic regime section at an oscilation frequency of 10 rad/s for 60 seconds. The horizontal gel strength was obtained by taking an average of the obtained storage modulus. For reference, the loss modulus was measured as a very small value as compared to the storage modulus.

**(5) Saline Flow Conductivity (SFC)**

The saline flow conductivity was measured in accordance with the method disclosed in Column 16 [0184] to [0189] of U.S. patent application publication no. 2009-0131255.

**Example 1**

A monomer aqueous solution having a monomer concentration of 45% by weight was prepared by mixing 100 g of acrylic acid, 0.5 of polyethylene glycol diacrylate (Mw=523) as a crosslinking agent, 83.3 g of 50% caustic soda (NaOH), and 89.8 g of water.

Subsequently, 810 g of the monomer aqueous solution was mixed with 30.54 g of a 0.18% ascorbic acid solution and 33 g of a 1% sodium persulfate solution, and the mixture was fed through a feed section of a continuous polymerization reactor with a kneader, together with 30.45 g of a 0.15% hydrogen peroxide solution, to thereby perform polymerization. At this time, the temperature of the polymerization device was maintained at 80°C, the maximum polymerization temperature was 110°C, and the polymerization time was 1 minute and 15 seconds(s). Thereafter, kneading was continuously performed, and polymerization and kneading were performed for 20 minutes. Thus, the resulting polymers were distributed so as to have a size of 0.2 cm or less. At this time, the water content of the hydrogel polymer finally formed was 51% by weight.

Subsequently, the hydrogel polymer was dried with a hot air dryer at 180°C for 30 minutes, and the dried hydrogel polymer was pulverized with a pin mill pulverization machine. Next, the polymer was classified into a polymer having a particle size of less than 150 $\mu$m and a polymer having a particle size of less than 150 $\mu$m to 850 $\mu$m by using a sieve.

Subsequently, a surface treatment solution containing 5 wt% of 1.3-propanediol and 5 wt% of propylene glycol was sprayed on the base polymer, stirred at room temperature and mixed so that the surface treatment solution is evenly distributed on the base polymer. Thereafter, such base polymer was added to the surface crosslinking reactor and subjected to a surface crosslinking reaction. Within such surface crosslinking reactor, the base polymer was confirmed to be gradually heated to the initial temperature in the vicinity of about 160°C, and after a lapse of about 30 minutes the reactor was operated to reach a maximum reaction temperature of about 185 °C. After reaching this maximum reaction temperature, an additional reaction was carried out for about 30 minutes and then a super absorbent polymer sample finally produced was taken. The surface crosslinking reaction conditions of Example 1 were summarized in Table 1 below. After the surface crosslinking process, the surface cross-linked super absorbent polymer having a particle size of about 150 to 850 $\mu$m was obtained using a sieve. The content of fine particles having a particle size of about 150 $\mu$m or less in the product of the super absorbent polymer was less than about 2 wt%.

**Examples 2-6**

The super absorbent polymers of Examples 2 to 6 were obtained in the same manner as in Example 1, except that, in the surface crosslinking reaction process, the surface cross-linking reaction conditions, such as the initial temperature of the base polymer during the reaction starting, the maximum reaction temperature, the time of temperature rise from the initial temperature to the maximum reaction temperature, and the holding time at the maximum reaction temperature, were changed as shown in Table 1 below.

**Comparative Examples 1-3**

The super absorbent polymers of Comparative Examples 1 to 3 were obtained in the same manner as in Example 1, except that, in the surface crosslinking reaction process, the surface cross-linking reaction conditions, such as the initial temperature of the base polymer during the reaction starting, the maximum reaction temperature, the time of temperature rise from the initial temperature to the maximum reaction temperature, and the holding time at the maximum reaction temperature, were changed as shown in Table 1 below.

[Table 1]

| | Gel strength before surface crosslinking(Pa) | Initial temperature during surface crosslinking reaction starting (°C) | Maximum reaction temperature (°C) | Time of temperature rise(min) | Holding time at maximum reaction temperature (min) |
|---|---|---|---|---|---|
| Example 1 | 4,800 | 160 | 185 | 30 | 30 |
| Example 2 | 4,800 | 160 | 185 | 30 | 20 |
| Example 3 | 4,800 | 160 | 185 | 30 | 40 |
| Example 4 | 4,800 | 160 | 190 | 20 | 25 |
| Example 5 | 4,800 | 160 | 190 | 20 | 30 |
| Example 6 | 4,800 | 160 | 180 | 40 | 45 |
| Comparative Example1 | 4,800 | 140 | 140 | 20 | 40 |
| Comparative Example2 | 4,800 | 160 | 185 | 30 | 10 |
| Comparative Example3 | 4,800 | 160 | 210 | 10 | 10 |

For the super absorbent polymers of Examples 1 to 6 and Comparative Examples 1 to 3, the physical property measurement and evaluation of CRC, AUP and gel strength (G') were carried out, and the measured physical property values are shown in Table 2 below. In addition, from the measured CRC, AUP and gel strength, the left hand values of the relationship represented by the following formula 1 were calculated and shown in Table 2 below.

[Formula 1]

$$\left(\frac{AUP}{CRC} \times \frac{G'}{1000}\right)^2 > 15\,Pa^2$$

[Table 2]

| | Example 1 | Example 2 | Example 3 | Example 4 | Example 5 | Example 6 | Comparative Example 1 | Comparative Example 2 | Comparative Example 3 |
|---|---|---|---|---|---|---|---|---|---|
| CRC(g/g) | 29.2 | 30.5 | 28.6 | 29.5 | 28.7 | 31.8 | 32.7 | 32.7 | 31.4 |
| AUP(g/g) | 25.0 | 25.4 | 24.1 | 24.6 | 23.8 | 23.9 | 22.1 | 21.2 | 21.8 |
| Gel strength (Pa) | 9309 | 8354 | 10248 | 8995 | 10200 | 7394 | 5033 | 4884 | 5292 |
| Left side of formula 1(Pa²) | 63.5 | 48.4 | 74.6 | 56.3 | 71.5 | 30.9 | 11.6 | 10.0 | 13.5 |

As shown in Table 2 above, it was confirmed that Examples 1 to 6 were generally excellent in CRC, AUP and gel strength and thus satisfied the relationship where the value of the formula 1 was 15 $Pa^2$ or more, whereas Comparative Examples 1 to 3 were poor in one or more of the above three physical properties and thus did not satisfy the relationship of the formula 1. In particular, in the case of Comparative Examples 1 to 3, the temperature rise condition and the reaction condition in the surface crosslinking process were different from those of Examples, and it is expected that Comparative Examples 1 to 3 exhibit poor gel strength or AUP as well as poor liquid permeability as compared to Examples.

### Example 7

A monomer aqueous solution having a monomer concentration of 45% by weight was prepared by mixing 100 g of acrylic acid, 0.5 g of polyethylene glycol diacrylate (Mw=523) as a crosslinking agent, 83.3 g of 50% caustic soda (NaOH), and 89.8 g of water.

Subsequently, 810 g of the monomer aqueous solution was mixed with 30.54 g of a 0.18% ascorbic acid solution and 33 g of a 1% sodium persulfate solution, and the mixture was fed through a feed section of a continuous polymerization reactor with a kneader, together with 30.45 g of a 0.15% hydrogen peroxide solution, to thereby perform polymerization. At this time, the temperature of the polymerization reactor was maintained at 80°C, the maximum polymerization temperature was 110°C, and the polymerization time was 1 minute and 15 seconds(s). Thereafter, kneading was continuously performed, and polymerization and kneading were performed for 20 minutes. Thus, the resulting polymers were distributed so as to have a size of 0.2 cm or less. At this time, the water content of the hydrogel polymer finally formed was 51% by weight.

Subsequently, the hydrogel polymer was dried with a hot air dryer at 180°C for 30 minutes, and the dried hydrogel polymer was pulverized with a pin mill pulverization machine. Next, the polymer was classified into a polymer having a particle size of less than about 150 $\mu$m and a polymer having a particle size of less than about 150 $\mu$m to 850 $\mu$m by using a sieve.

Subsequently, a surface treatment solution containing 5 wt% of 1.3-propanediol and 5 wt% of propylene glycol was sprayed on the base polymer, stirred at room temperature and mixed so that the surface treatment solution is evenly distributed on the base polymer. Thereafter, such base polymer was added to the surface crosslinking reactor and subjected to a surface crosslinking reaction. Within such surface crosslinking reactor, the base polymer was confirmed to be gradually heated to the initial temperature in the vicinity of about 60°C, and after a lapse of about 15 minutes, the reactor was operated to reach a maximum reaction temperature of about 185°C. After reaching this maximum reaction temperature, an additional reaction was carried out for about 30 minutes and then a super absorbent polymer sample finally produced was taken. The surface crosslinking reaction conditions of Example 7 were summarized in Table 3 below. After the surface crosslinking process, the surface cross-linked super absorbent polymer having a particle size of about 150 to 850 $\mu$m was obtained using a sieve. The content of fine particles having a particle size of about 150 $\mu$m or less in the product of the super absorbent polymer was less than about 1 wt%.

### Examples 8, 9 and Comparative Example 4

The super absorbent polymers were prepared in the same manner as in Example 7, except that the reaction time during surface cross-linking reaction, the maximum reaction temperature and the temperature rise rate were changed as shown in Table 3 below.

### Examples 10

The base polymer was prepared by proceeding up to the classifying process in the same manner as in Example 7, except that 0.8 g of polyethylene glycol diacrylate (Mw=523) was used as an internal crosslinking agent.

And, the time required to reach a maximum reaction temperature of about 185□ at the initial temperature during surface crosslinking reaction was operated to 15 minutes. After reaching this maximum reaction temperature, an additional reaction was carried out for about 20 minutes and then a super absorbent polymer sample finally produced was taken. The surface crosslinking reaction conditions of Example 10 were summarized in Table 3 below. After the surface crosslinking process, the surface cross-linked super absorbent polymer having a particle size of about 150 to 850 $\mu$m was obtained using a sieve. The content of fine particles having a particle size of about 150 $\mu$m or less in the product of the super absorbent polymer was less than about 1 wt%.

### Comparative Examples 5

The base polymer was prepared by proceeding up to the classifying process in the same manner as in Example 7, except that 0.8 g of polyethylene glycol diacrylate (Mw=523) was used as an internal crosslinking agent.

And, the time required to reach a maximum reaction temperature of about 180□ at the initial temperature during surface crosslinking reaction was operated to 30 minutes. After reaching this maximum reaction temperature, a further reaction was carried out for about 10 minutes and then a super absorbent polymer sample finally produced was taken. The surface crosslinking reaction conditions of Comparative Example 5 were summarized in Table 3 below. After the surface crosslinking process, the surface cross-linked super absorbent polymer having a particle size of about 150 to 850 μm was obtained using a sieve.

**Comparative Examples 6**

The base polymer was prepared by proceeding up to the classifying process in the same manner as in Example 7, except that 0.2 g of polyethylene glycol diacrylate (Mw=523) was used as an internal crosslinking agent.
And, the time required to reach a maximum reaction temperature of about 180□ at the initial temperature during surface crosslinking reaction was operated to 15 minutes. After reaching this maximum reaction temperature, an additional reaction was carried out for about 20 minutes and then a super absorbent polymer sample finally produced was taken. The surface crosslinking reaction conditions of Comparative Example 6 were summarized in Table 3 below. After the surface crosslinking process, the surface cross-linked super absorbent polymer having a particle size of about 150 to 850 μm was obtained using a sieve.

[Table 3]

|  | Gel strength before surface crosslinking(Pa) | Initial temperature during surface crosslinking reaction starting (°C) | Maximum reaction temperature (°C) | Time of temperature rise(min) | Holding time at maximum reaction temperature (min) |
|---|---|---|---|---|---|
| Example 7 | 4,800 | 60 | 185 | 15 | 30 |
| Example 8 | 4,800 | 60 | 185 | 15 | 20 |
| Example 9 | 4,800 | 60 | 185 | 15 | 50 |
| Example 10 | 5,700 | 60 | 185 | 15 | 20 |
| Comparative Example 4 | 4,800 | 60 | 185 | 15 | 10 |
| Comparative Example 5 | 5,700 | 60 | 180 | 30 | 10 |
| Comparative Example 6 | 3,400 | 60 | 185 | 15 | 20 |

The physical properties of Examples 7 to 10 and Comparative Examples 4 to 6, measured by the method described above, were summarized in Table 4 below. And, from the measured CRC, AUP, G' and SFC, the left hand values of the relationship of the following formulae 1 and 2 were calculated and shown in Table 4 below.

* Formula 1:

$$\left( \frac{AUP}{CRC} \times \frac{G'}{1000} \right)^2 > 15\,Pa^2$$

* Formula 2: $[(AUP+CRC)/2] * [G'/1000] * SFC > 100$

[Table 4]

| | CRC (g/g) | AUP (g/g) | G' (Pa) | SFC ($\cdot 10^{-7}$ cm$^3\cdot$s/g) | Left side value of formula 1(Pa$^2$) | Left side value of formula 2 |
|---|---|---|---|---|---|---|
| Example 7 | 29.0 | 23.7 | 10402 | 73 | 72.3 | 141.5 |
| Example 8 | 29.7 | 24.8 | 9767 | 50 | 66.5 | 115.4 |
| Example 9 | 26.2 | 22.6 | 11028 | 70 | 90.5 | 137.2 |
| Example 10 | 29.5 | 24.5 | 9840 | 54 | 66.8 | 119.8 |
| Comparative Example 4 | 30.5 | 21.4 | 5354 | 27 | 14.1 | 61.2 |
| Comparative Example 5 | 29.7 | 19.2 | 5770 | 35 | 13.9 | 70.3 |
| Comparative Example 6 | 32.8 | 18.3 | 4183 | 15 | 5.4 | 40.0 |

As shown in Table 4 above, it was confirmed that the super absorbent polymers obtained in Examples were generally excellent in CRC, AUP, G' and SFC and thus not only satisfied the relationship where the value of the formula 1 was 15 Pa$^2$ or more but also exhibited the relationship where the value of the formula 2 was 100 or more, whereas Comparative Examples 4 to 6 were poor in one or more of the above physical properties and thus did not satisfy the relationship of the formulae 1 and 2. In particular, in the case of Comparative Examples 4 to 6, the gel strength before surface crosslinking, the temperature rise condition in the surface crosslinking process, or the reaction condition (the holding time at the maximum reaction temperature, etc.) were different from those of Examples, and it was confirmed that Comparative Examples 4 to 6 were poor in G' or SFC and thus poor in liquid permeability as compared to Examples.

## Claims

1. A super absorbent polymer comprising a cross-linked polymer in which a base polymer in the form of a powder obtained by polymerizing water-soluble ethylene-based unsaturated monomers having an acidic group in which at least a part thereof is neutralized is subjected to a surface crosslinking, wherein the super absorbent polymer satisfies the relationship represented by the following formula 1:

wherein the super absorbent polymer has CRC of 26 to 32 g/g, AUP of 22 to 26 g/g, a horizontal gel strength G' of 7,000 to 12,000 Pa, and SFC of 50 to 75 ($\cdot 10^{-7}$cm$^3\cdot$s/g),
wherein the crosslinked polymer is obtained by cross-linking the surface of the base polymer in the presence of a surface crosslinking agent containing diol or polyol having 2 to 8 carbon atoms,
wherein the base polymer has a gel strength before surface crosslinking (G'; Pa) of 4500Pa or more, and
wherein the surface crosslinking reaction is performed by heating the surface crosslinking agent-added base polymer particles at 180 to 200°C for at least 20 minutes, the maximum reaction temperature of the surface crosslinking reaction is 180 to 200 °C, and the holding time at the maximum reaction temperature is 20 minutes to 50 minutes:

[Formula 1]

$$\left( \frac{AUP}{CRC} \times \frac{G'}{1000} \right)^2 > 15 \, Pa^2$$

in the formula 1,

CRC represents a centrifuge retention capacity for a physiological saline solution (0.9 wt% aqueous sodium chloride solution) of the super absorbent polymer for 30 minutes, and is measured in accordance with EDANA (European Disposables and Nonwovens Association) recommended test method No. WSP 241.3,

AUP represents an absorbency under pressure for a physiological saline solution (0.9 wt% aqueous sodium chloride solution) of the super absorbent polymer under 0.7 psi for 1 hour, and is measured in accordance with EDANA recommended test method No. WSP 242.3, and

G' represents a horizontal gel strength of the super absorbent resin measured using a rheometer, after absorbing and swelling a physiological saline solution (0.9 wt% aqueous sodium chloride solution) to the super absorbent polymer for 1 hour,

wherein the horizontal gel strength G' of the super absorbent resin is measured by a method as described in the specification, comprising the steps of:

absorbing a physiological saline solution to the super absorbent polymer to swell the super absorbent polymer; positioning the swelled super absorbent polymer between plates of a rheometer having a predetermined interval to pressurize the two plate surfaces; confirming a shear strain in the linear viscoelastic regime section where storage modulus and loss modulus are steady, while increasing the shear strain using the rheometer under vibration; and measuring the storage modulus and the loss modulus of the swelled super absorbent polymer under the confirmed shear strain, respectively, and measuring the average value of the storage modulus as a gel strength, and

wherein the SFC represents a physiological saline solution (0.685 wt% aqueous sodium chloride solution) flow conductivity ($\cdot 10^{-7} cm^3 \cdot s/g$) for the super absorbent polymer, and is measured in accordance with the method disclosed in the specification.

2. The super absorbent polymer according to Claim 1 wherein the super absorbent polymer satisfies the relationship represented by the following formula 2.

[Formula 2]

$$\sqrt{\frac{(CRC+AUP)}{2} * \left(\frac{G'}{1000}\right) *SFC} > 100.$$

in the formula 2, AUP, CRC and G' are as defined in the formula 1 and SFC represents a physiological saline solution (0.685 wt% aqueous sodium chloride solution) flow conductivity ($\cdot 10^{-7} cm^3 \cdot s/g$) for the super absorbent polymer, and is measured in accordance with the method disclosed in the specification.

3. The super absorbent polymer according to Claim 1 wherein the water-soluble ethylene-based unsaturated monomer includes one or more selected from the group consisting of an anionic monomer such as acrylic acid, methacrylic acid, maleic anhydride, fumaric acid, crotonic acid, itaconic acid, 2-acryloyl ethane sulfonic acid, 2-methacryloyl ethane sulfonic acid, 2-(meth)acryloyl propane sulfonic acid, or 2-(meth)acrylamide-2-methylpropane sulfonic acid, and a salt thereof; a nonionic hydrophilic monomer such as (meth)acrylamide, N-substituted (meth)acrylate, 2-hydroxyethyl(meth)acrylate, 2-hydroxypropyl(meth)acrylate, methoxypolyethyleneglycol(meth)acrylate, or polyethyleneglycol(meth)acrylate; and an unsaturated monomer containing amino group such as (N,N)-dimethylaminoethyl(meth)acrylate or (N,N)-dimethylaminopropyl(meth)acrylate, and a quaternary compound thereof.

4. The super absorbent polymer according to Claim 1 wherein the base polymer includes a polymer wherein the above monomer is cross-linked and polymerized in the presence of one or more internal crosslinking agents selected from the group consisting of bis(meth)acrylamide having 8 to 12 carbon atoms, poly(meth)acrylate of polyol having 2 to 10 carbon atoms and poly(meth)allyl ether of polyol having 2 to 10 carbon atoms.

5. The super absorbent polymer according to Claim 1 wherein the super absorbent polymer has a particle size of 150 to 850μm, as measured by EDANA recommended test method No. WSP 220.3.

6. Method for preparing a superabsorbent polymer comprising a cross-linked polymer according to claim 1, comprising the steps:

obtaining a base polymer by polymerizing water-soluble ethylene-based unsaturated monomers having an acidic group in which at least a part thereof is neutralized,

subjecting the base polymer in the form of a powder to a surface crosslinking, wherein the surface crosslinking reaction is performed by heating the surface crosslinking agent-added base polymer particles at 180 to 200°C for at least 20 minutes, the maximum reaction temperature

of the surface crosslinking reaction is 180 to 200 °C, and the holding time at the maximum reaction temperature is 20 minutes to 50 minutes,

wherein the surface crosslinking agent contains diol or polyol having 2 to 8 carbon atoms, and the base polymer has a gel strength before surface crosslinking (G'; Pa) of 4500Pa or more.

**Patentansprüche**

1. Superabsorbierendes Polymer, das ein vernetztes Polymer umfasst, indem ein Basispolymer in der Form eines Pulvers, das mittels Polymerisation von wasserlöslichen, auf Ethylen basierten, ungesättigten Monomeren mit einer Säuregruppe, in der mindestens ein Teil davon neutralisiert ist, erhalten wird, einer Oberflächenvernetzung ausgesetzt wird, wobei das superabsorbierende Polymer dem Verhältnis genügt, das durch die folgende Formel 1 dargestellt ist,

wobei das superabsorbierende Polymer eine CRC von 26 bis 32 g/g, eine AUP von 22 bis 26 g/g, eine horizontale Gelstärke G' von 7000 bis 12000 Pa und eine SFC von 50 bis 75 ($\cdot 10^{-7}$ cm$^3$$\cdot$s/g) aufweist,

wobei das vernetzte Polymer erhalten wird, indem die Oberfläche des Basispolymers in der Gegenwart eines Oberflächenvernetzers vernetzt wird, der Diol oder Polyol mit 2 bis 8 Kohlenstoffatomen enthält,

wobei das Basispolymer vor der Oberflächenvernetzung eine Gelstärke (G'; Pa) von 4500 Pa oder mehr aufweist, und wobei die Oberflächenvernetzungsreaktion erfolgt, indem Basispolymerpartikel, denen Oberflächenvernetzer hinzugefügt worden ist, bei 180 bis 200 °C für mindestens 20 Minuten erwärmt werden, wobei die maximale Reaktionstemperatur der Oberflächenvernetzungsreaktion 180 bis 200 °C beträgt und die Haltezeit bei der maximalen Reaktionstemperatur 20 Minuten bis 50 Minuten beträgt:

$$\left(\frac{AUP}{CRC} \times \frac{G\prime}{1000}\right)^2 > 15 Pa^2 \qquad [\text{Formel 1}]$$

in der Formel 1,

stellt CRC eine Zentrifugenretentionskapazität für eine physiologische Salzlösung (0,9 Gew.-% wässrige Natriumchloridlösung) des superabsorbierenden Polymers für 30 Minuten dar und wird in Übereinstimmung mit der von der EDANA (Europäische Einwegartikel- und Vliesstoff-Vereinigung) empfohlenen Testmethode Nr. WSP 241.3 gemessen,

stellt AUP ein Absorptionsvermögen unter Druck für eine physiologische Salzlösung (0,9 Gew.-% wässrige Natriumchloridlösung) des superabsorbierenden Polymers unter 48 hPa (0,7 psi) für eine Stunde dar und wird in Übereinstimmung mit der von der EDANA empfohlenen Testmethode Nr. WSP 242.3 gemessen und

stellt G' eine horizontale Gelstärke des superabsorbierenden Harzes dar, die unter Verwendung eines Rheometers gemessen wird, nachdem eine physiologische Salzlösung (0,9 Gew.-% wässrige Natriumchloridlösung) durch das superabsorbierende Polymer für eine Stunde absorbiert wurde und dieses gequollen ist,

wobei die horizontale Gelstärke G' des superabsorbierenden Harzes mittels einer Methode gemessen wird, die in der Beschreibung beschrieben ist, und die Schritte umfasst, dass

eine physiologische Salzlösung von dem superabsorbierenden Polymer absorbiert wird, um das superabsorbierende Polymer aufzuquellen, das gequollene superabsorbierende Polymer zwischen den Platten eines Rheometers positioniert wird, das eine vorbestimmten Intervall aufweist, um die beiden Plattenoberflächen unter Druck zu setzen, eine Scherspannung in dem linearen viskoelastischen Systembereich verifiziert wird, in dem das Speichermodul und das Verlustmodul stetig sind, während die Scherspannung unter Verwendung des Rheometers unter Vibration erhöht wird, und das Speichermodul und das Speichermodul des gequollenen superabsorbierenden Polymers unter der verifizierten Scherspannung jeweils gemessen werden und der Mittelwert des Speichermoduls als eine Gelstärke gemessen wird, und

wobei die SFC eine Flussleitfähigkeit ($\cdot 10^{-7}$ cm$^3$$\cdot$s/g) einer physiologischen Salzlösung (0,685 Gew.-% wässrige Natriumchloridlösung) für das superabsorbierende Polymer darstellt und in Übereinstimmung mit der in der Beschreibung offenbarten Methode gemessen wird.

2. Superabsorbierendes Polymer nach Anspruch 1, bei dem das superabsorbierende Polymer dem Verhältnis genügt, das durch die folgende Formel 2 dargestellt ist.

$$\sqrt{\frac{(CRC+AUP)}{2} * \left(\frac{G\prime}{1000}\right) * SFC} > 100 \qquad [\text{Formel } 2]$$

in der Formel 2 sind AUP, CRC und G' wie in Formel 1 definiert, und SFC stellt eine Flussleitfähigkeit ($\cdot10^{-7}$ cm$^3\cdot$s/g) einer physiologische Salzlösung (0,685 Gew.-% wässrige Natriumchloridlösung) für das superabsorbierende Polymer dar und wird in Übereinstimmung mit der in der Beschreibung offenbarten Methode gemessen.

3. Superabsorbierendes Polymer nach Anspruch 1, bei dem das wasserlösliche, auf Ethylen basierte, ungesättigte Monomer eines oder mehrere ausgewählt aus der Gruppe bestehend aus einem anionischen Monomer, wie z.B. Acrylsäure, Methacrylsäure, Maleinsäure, Fumarsäure, Crotonsäure, Itaconsäure, 2-Acryloylethansulfonsäure, 2-Methacryloylethansulfonsäure, 2-(Meth)acryloylpropansulfonsäure oder 2-(Meth)acrylamid-2-methylpropansulfonsäure und einem Salz davon, einem nicht-ionischen hydrophilen Monomer, wie z.B. (Meth)acrylamid, N-substituiertem (Meth)acrylat, 2-Hydroxyethyl(meth)acrylat, 2-Hydroxypropyl(meth)acrylat, Methoxypolyethylenglycol(meth)acrylat oder Polyethylenglycol(meth)acrylat, und einem ungesättigten Monomer, das Aminogruppen enthält,, wie z.B. (N,N)-Dimethylaminoethyl(meth)acrylat oder (N,N)-Dimethylaminopropyl(meth)acrylat und eine quarternäre Verbindung davon, umfasst.

4. Superabsorbierendes Polymer nach Anspruch 1, bei dem das Basispolymer ein Polymer umfasst, bei dem das obige Monomer in der Gegenwart von einem oder mehreren internen Vernetzern ausgewählt aus der Gruppe bestehend aus Bis(meth)acrylamid mit 8 bis 12 Kohlenstoffatomen, Poly(meth)acrylat aus Polyol mit 2 bis 10 Kohlenstoffatomen und Poly(meth)allylether aus Polyol mit 2 bis 10 Kohlenstoffatomen vernetzt und polymerisiert wird.

5. Superabsorbierendes Polymer nach Anspruch 1, bei dem das superabsorbierende Polymer eine Partikelgröße von 150 bis 850 µm aufweist, die mittels der von der EDANA empfohlenen Testmethode Nr. WSP 220.3 gemessen wird.

6. Verfahren zur Herstellung eines superabsorbierenden Polymers, das ein vernetztes Polymer gemäß Anspruch 1 umfasst, das die Schritt umfasst, dass
ein Basispolymer erhalten wird, indem wasserlösliche, auf Ethylen basierte, ungesättigte Monomere polymerisiert werden, die eine Säuregruppe aufweisen, von der mindestens ein Teil neutralisiert ist,
das Basispolymer in der Form eines Pulvers einer Oberflächenvernetzung ausgesetzt wird, wobei die Oberflächenvernetzungsreaktion durchgeführt wird, indem Basispolymerpartikel, denen Oberflächenvernetzer hinzugefügt wurde, bei 180 bis 200 °C für mindestens 20 Minuten erwärmt werden, wobei die maximale Reaktionstemperatur der Oberflächenvernetzungsreaktion 180 bis 200 °C beträgt und die Haltezeit der maximalen Reaktionstemperatur 20 Minuten bis 50 Minuten beträgt,
wobei der Oberflächenvernetzer Diol oder Polyol mit 2 bis 8 Kohlenstoffatomen enthält und das Basispolymer vor der Oberflächenvernetzung eine Gelstärke (G'; Pa) von 4500 Pa oder mehr aufweist.

## Revendications

1. Polymère superabsorbant comprenant un polymère réticulé dans lequel un polymère de base sous la forme d'une poudre obtenu par polymérisation de monomères à base d'éthylène insaturés et hydrosolubles portant un groupement acide dont une partie au moins est neutralisée est soumis à une réticulation en surface, où le polymère superabsorbant satisfait la relation représentée par la formule 1 ci-dessous :

où le polymère superabsorbant a une capacité d'absorption de l'eau après centrifugation (CRC) qui va de 26 à 32 g/g, une absorbance sous pression (AUP) qui va de 22 à 26 g/g, une résistance de gel en direction horizontale G' qui va de 7 000 à 12 000 Pa et une conductivité du flux d'une solution saline physiologique (SFC) qui va de 50 à 75($\cdot10^{-7}$cm$^3\cdot$s/g),
où le polymère réticulé est obtenu par réticulation en surface du polymère de base en la présence d'un agent de réticulation en surface contenant un diol ou un polyol à 2 à 8 atomes de carbone,
où le polymère de base a une résistance de gel avant réticulation en surface (G' ; Pa) égale ou supérieure à 4 500 Pa et
où la réaction de réticulation en surface est effectuée en chauffant des particules du polymère de base auxquelles l'agent de réticulation en surface est ajouté à 180 à 200 °C pendant au moins 20 minutes, la température maximale de la réaction de réticulation en surface allant de 180 à 200 °C et le temps de maintien à la température maximale de réaction allant de 20 minutes à 50 minutes :

[Formule 1]

$$\left(\frac{AUP}{CRC} \times \frac{G'}{1000}\right)^2 > 15\ Pa^2$$

À la formule 1,

CRC représente une capacité de rétention de fluides en solution saline physiologique (solution aqueuse de chlorure de sodium à 0,9 % en poids) après centrifugation du polymère superabsorbant pendant 30 minutes, mesurée conformément à la méthode d'essai recommandée N° WSP 241.3 de l'EDANA (*European Disposables and Nonwovens Association*),

AUP représente l'absorbance sous pression pour une solution saline physiologique (solution aqueuse de chlorure de sodium à 0,9 % en poids) du polymère superabsorbant sous une pression de 0,7 psi pendant 1 heure, mesurée conformément à la méthode d'essai recommandée N° WSP 242.3 de l'EDANA et

G' représente une résistance de gel en direction horizontale de la résine superabsorbante, mesurée au moyen d'un rhéomètre après absorption d'une solution saline physiologique (solution aqueuse de chlorure de sodium à 0,9 % en poids) par le polymère superabsorbant et gonflement de celui-ci pendant 1 heure,

où la résistance de gel en direction horizontale G' de la résine superabsorbante est mesurée par une méthode telle que décrite dans la spécification qui comprend les étapes consistant à :

absorber une solution saline physiologique avec le polymère superabsorbant pour faire gonfler le polymère superabsorbant ; placer le polymère superabsorbant gonflé entre les plaques d'un rhéomètre séparées par un intervalle prédéterminé pour pressuriser les surfaces des deux plaques ; confirmer une déformation de cisaillement, dans le comportement viscoélastique linéaire, à laquelle le module d'accumulation et le module de dissipation sont stables tout en augmentant la déformation de cisaillement en utilisant le rhéomètre sous vibration ; et mesurer le module d'accumulation et le module de dissipation, respectivement, du polymère superabsorbant gonflé à la déformation de cisaillement confirmée et mesurer la valeur moyenne du module d'accumulation en tant que résistance de gel et

où SFC représente la conductivité de flux ($\cdot 10^{-7} cm^3 \cdot s/g$) d'une solution saline physiologique (solution aqueuse de chlorure de sodium à 0,685 % en poids) du polymère superabsorbant, mesurée conformément à la méthode divulguée dans la spécification.

2. Polymère superabsorbant selon la revendication 1, où le polymère superabsorbant satisfait la relation représentée par la formule 2 suivante :

[Formule 2]

$$\sqrt{\frac{(CRC+AUP)}{2} * \left(\frac{G'}{1000}\right) * SFC} > 100.$$

À la formule 2, AUP, CRC et G' sont tels que définis à la formule 1 et SFC représente la conductivité du flux ($\cdot 10^{-7} cm^3 \cdot s/g$) d'une solution saline physiologique (solution aqueuse de chlorure de sodium à 0,685 % en poids) du polymère superabsorbant, mesurée conformément à la méthode divulguée dans la spécification.

3. Polymère superabsorbant selon la revendication 1, où le monomère à base d'éthylène insaturé et hydrosoluble inclut un ou plusieurs sélectionnés dans le groupe consistant en un monomère anionique comme l'acide acrylique, l'acide méthacrylique, l'anhydride maléique, l'acide fumarique, l'acide crotonique, l'acide itaconique, l'acide 2-acryloyl-éthane sulfonique, l'acide 2-méthacryloyl-éthane sulfonique, l'acide 2-(méth)acryloyl-propane sulfonique ou l'acide 2-(méth)acrylamide-2-méthylpropane sulfonique et un sel de ceux-ci ; un monomère hydrophile non ionique comme le (méth)acrylamide, un (méth)acrylate N-substitué, le (méth)acrylate de 2-hydroxyéthyle, le (méth)acrylate de 2-hydroxypropyle, le (méth)acrylate de méthoxy(polyéthylène glycol) ou le (méth)acrylate de polyéthylène glycol ; et un monomère insaturé contenant un groupement amino comme le (méth)acrylate de (N,N)-diméthylaminoéthyle ou le (méth)acrylate de (N,N)-diméthylaminopropyle et un composé quaternaire de ceux-ci.

4. Polymère superabsorbant selon la revendication 1, où le polymère de base inclut un polymère dans lequel le monomère susmentionné est réticulé et polymérisé en la présence de un ou plusieurs agents de réticulation internes sélectionnés dans le groupe consistant en un bis(méth)acrylamide à 8 à 12 atomes de carbone, un poly[(méth)acrylate de polyol] à 2 à 10 atomes de carbone et un poly[éther (méth)allylique de polyol] à 2 à 10 atomes de carbone.

5. Polymère superabsorbant selon la revendication 1, où le polymère superabsorbant a une dimension particulaire, telle que mesurée conformément à la méthode d'essai recommandée N° WSP 220.3 de l'EDANA, qui va de 150 à 850 $\mu$m.

6. Procédé de préparation d'un polymère superabsorbant comprenant un polymère réticulé selon la revendication 1, comprenant les étapes consistant à :

   obtenir un polymère de base par polymérisation de monomères à base d'éthylène insaturés et hydrosolubles portant un groupement acide dont une partie au moins est neutralisée,
   soumettre le polymère de base sous la forme d'une poudre à une réticulation en surface, où la réaction de réticulation en surface est effectuée en chauffant des particules du polymère de base auxquelles l'agent de réticulation en surface est ajouté à 180 à 200 °C pendant au moins 20 minutes, la température maximale de la réaction de réticulation en surface allant de 180 à 200 °C et le temps de maintien à la température maximale de réaction allant de 20 minutes à 50 minutes,
   où l'agent de réticulation en surface contient un diol ou un polyol à 2 à 8 atomes de carbone et où le polymère de base a une résistance de gel avant réticulation en surface (G' ; Pa) égale ou supérieure à 4 500 Pa.

**REFERENCES CITED IN THE DESCRIPTION**

**Patent documents cited in the description**

- KR 1020040058305 A
- KR 20030068198 A
- US 6951911 A
- WO 2005097313 A1
- WO 0191815 A2
- EP 1770113 A1
- KR 1020140130034 A
- EP 3020737 A1
- US 20090131255

**Non-patent literature cited in the description**

- **REINHOLD SCHWALM.** UV Coatings: Basics, Recent Developments and New Application. Elsevier, 2007, 115
- **ODIAN.** Principle of Polymerization. Wiley, 1981, 203